# EUROPEAN PATENT APPLICATION

(11) **EP 4 104 825 A1**
(43) Date of publication of application: **21.12.2022**
(21) Application number: 21753798.4
(22) Date of filing: 15.02.2021
(51) Int. Cl.: A61K 31/17, A61K 9/00, A61K 45/06, A61K 31/00, A61P 29/00, A61K 31/155, A61P 31/04

(54) **PHARMACEUTICAL COMPOSITION FOR INHIBITING INFLAMMATORY RESPONSE COMPRISING HYDROXYUREA**

(30) Priority: 14.02.2020 KR 20200018534; 10.04.2020 KR 20200044119
(71) Applicant: Bionoxx Inc., Seongnam-si, Gyeonggi-do 13554 (KR)
(72) Inventor: HWANG, Tae-Ho, Yangsan-si, Gyeongsangnam-do 50612 (KR); CHO, Mong, Yangsan-si, Gyeongsangnam-do 50612 (KR); CHO, Euna, Yangsan-si, Gyeongsangnam-do 50612 (KR); LEE, Bora, Yangsan-si, Gyeongsangnam-do 50612 (KR); KIM, Eung-Kyun, Yangsan-si, Gyeongsangnam-do 50612 (KR); LEE, Chan Hee, Yangsan-si, Gyeongsangnam-do 50612 (KR)
(74) Representative: Dehns
(86) International application number: PCT/KR2021/001910
(87) International publication number: WO 2021/162525

(57) **Abstract**

The present invention relates to use of hydroxyurea for preventing, alleviating, or treating systemic inflammatory response syndrome or sepsis. Hydroxyurea, which is the active ingredient of a pharmaceutical composition of the present invention, can control the activity of neutrophils, which are immune cells involved in inflammatory mechanisms in an individual, and thus can protect an individual from side effects or conditions caused by systemic inflammatory responses which can occur due to exposure to microorganisms such as bacteria or viruses. Thus, when the pharmaceutical composition of the present invention is administered to an individual suffering from a systemic inflammatory response, the pharmaceutical composition prevents, alleviates, or treats systemic inflammatory response syndrome or sepsis in the individual and ultimately can remarkably increase the survival rate of the individual. In addition, the composition according to the present invention can be effective in preventing, alleviating, or treating neutrophilia which can occur when exposed to microorganisms such as bacteria or viruses, wherein the viruses include viruses for therapeutic purposes, such as oncolytic viruses, as well as pathogenic viruses such as influenza virus or coronavirus.

## Description

### Technical Field

The present invention relates to a pharmaceutical use of hydroxyurea, which modulates immune cells such as neutrophils and thus exerts an effect of inhibiting an inflammatory response such as systemic inflammatory response syndrome or sepsis.

### Background Art

Systemic inflammatory response syndrome (SIRS) refers to a condition in which a severe inflammatory response occurs throughout the body. Clinically, a case where two or more of the following symptoms are observed is defined as systemic inflammatory response syndrome: hyperthermia with a body temperature of 38°C or higher or hypothermia with a body temperature of 36°C or lower, increased respiratory rate of over 24 breaths per minute (tachypnea), heart rate of over 90 beats per minute (tachycardia), and highly elevated or decreased white blood cell count on blood test. A systemic inflammatory response may be induced by sepsis, trauma, laceration, pancreatitis, or the like. In particular, in a case where this systemic inflammatory response syndrome is caused by microbial infection, it is called sepsis.

Sepsis refers to symptoms caused by immune responses that are induced throughout the body against infection resulting from an injury to a tissue or organ and are strong enough to threaten life. Sepsis is caused by an inflammatory immune response against infection. In general, such infection is caused by bacteria; however, sepsis may also be caused by infection with spores, viruses, or the like. Sepsis is caused by primary infection that occurs through organs such as the lungs, brain, urethra, skin, and abdomen. People, such as those who are too young or too old and those whose immunity has been weakened due to cancer or diabetes, may be susceptible to sepsis. Sepsis may cause a severe inflammatory response throughout the body, and thus result in irreversible damage to the body. Sepsis has mortality of 30%, and 30 million sepsis patients occur worldwide annually.

Such sepsis is mainly treated through supply of fluids and administration of antibiotics. In general, it is better to apply antibiotics as soon as possible; and in a case where supply of fluids is insufficient to maintain blood pressure, drugs may be used which can raise blood pressure. In addition, various anti-inflammatory substances and immunomodulators have been tried as therapeutic agents to inhibit hyperinflammatory responses in sepsis. Specifically, attempts have been made to treat sepsis using immunomodulators such as corticosteroids, anti-endotoxin antibodies, TNF antagonists, and IL-1 receptor antagonists; however, results showing amelioration of sepsis have not yet been obtained.

### Disclosure of Invention

### Technical Problem

Research has been conducted to treat sepsis; however, the drugs developed so far have insufficient therapeutic effects or have side effects. Thus, there is a need to develop a safe and effective therapeutic agent for sepsis. Accordingly, an object of the present invention is to provide a pharmaceutical composition for preventing, alleviating, or treating systemic inflammatory response syndrome or sepsis in a manner that inhibits a systemic inflammatory response.

### Solution to Problem

In order to solve the above-mentioned problem, in an aspect of the present invention, there is provided a pharmaceutical composition for preventing, alleviating, or treating systemic inflammatory response syndrome or sepsis, comprising hydroxyurea as an active ingredient.

### Advantageous Effects of Invention

Hydroxyurea, which is an active ingredient of the pharmaceutical composition of the present invention, can regulate the activity of neutrophils, which are immune cells involved in the inflammatory mechanism in an individual, and thus can protect an individual from side effects or pathological conditions caused by a systemic inflammatory response which may be induced by exposure to a microorganism such as bacterium or virus. Thus, in a case where the pharmaceutical composition of the present invention is administered to an individual suffering from a systemic inflammatory response, it is possible to prevent, alleviate, or treat systemic inflammatory response syndrome or sepsis in the individual, so that survival of the individual can be eventually remarkably increased. In addition, the composition according to the present invention can be effective in preventing, alleviating, or treating neutrophilia that may occur when exposed to a microorganism such as bacterium or virus, wherein the virus include viruses for therapeutic purposes, such as oncolytic viruses, as well as pathogenic viruses such as influenza virus or coronavirus.

### Brief Description of Drawings

FIG. 1 illustrates an experimental schedule for identifying an effect of hydroxyurea (HU) in a bacteria-induced sepsis model. Here, HU indicates a group having received HU once a day, and HU" indicates a group having received HU twice a day. This also applies to the rest of the drawings.
FIG. 2 illustrates results obtained by identifying survival, following treatment with HU, in mice with sepsis induced by bacterial lipopolysaccharide (LPS).
FIG. 3 illustrates results obtained by identifying survival, following treatment with HU, in mice with sepsis induced by bacterial LPS.
FIG. 4 illustrates results obtained by identifying survival, following treatment with HU, in mice with sepsis induced by the Western Reserve strain of vaccinia virus (WR virus, 1 × 10⁵ pfu).
FIG. 5 illustrates results obtained by inducing sepsis in mice using the Western Reserve strain of vaccinia virus, subjecting the mice to treatment with HU, and then measuring the number of virus particles in blood.
FIG. 6 illustrates results obtained by identifying survival, following treatment with HU, in mice with sepsis induced by the Western Reserve strain of vaccinia virus (1 × 10⁵ pfu and 1 × 10⁷ pfu).
FIG. 7 illustrates photographs taken after subjecting mice with sepsis induced by the Western Reserve strain of vaccinia virus to administration of HU or G-CSF, and then performing staining of liver and lung tissues.
FIG. 8 illustrates an experimental schedule for identifying an effect of HU in an influenza virus-induced sepsis model.
FIG. 9 illustrates results obtained by identifying survival, following treatment with Tamiflu and/or HU, in mice with sepsis induced by influenza virus.
FIG. 10 illustrates results obtained by observing changes in body weight, following treatment with Tamiflu and/or HU, in mice with sepsis induced by influenza virus.
FIG. 11 illustrates results obtained by performing an experiment for identifying whether HU has an antiviral effect. Here, CC₅₀ represents a concentration at which about 50% of the cells exhibit a cytotoxic response; and EC₅₀ represents a concentration at which about 50% of the drug effect is seen.
FIG. 12 illustrates photographs, showing levels of skin rashes and pustules, following treatment with HU, in monkeys with systemic inflammatory syndrome induced by the Western Reserve strain of vaccinia virus.
FIG. 13 illustrates results obtained by observing changes in body temperature and body weight, following treatment with HU, in monkeys with systemic inflammatory syndrome induced by the Western Reserve strain of vaccinia virus.
FIG. 14 illustrates results obtained by subjecting monkeys with systemic inflammatory syndrome induced by the Western Reserve strain of vaccinia virus to treatment with HU, and then measuring the number of virus particles in blood.
FIG. 15 illustrates results obtained by identifying changes in absolute neutrophil count (ANC), whole blood cell (WBC) count, and absolute lymphocyte count (ALC), following treatment with HU, in monkeys with systemic inflammatory syndrome induced by the Western Reserve strain of vaccinia virus.
FIG. 16 illustrates results, showing that in a case of being infected with various types of viruses including SARS-CoV-2, a high level of absolute neutrophil count (ANC) is observed in patients with "Fatal" as compared with patients with "Non-Fatal".
FIG. 17 illustrates results obtained by analyzing blood samples of patients who died early upon oncolytic virus infection, showing that the ANC increased rapidly in the patients who died early.
FIG. 18 illustrates results obtained by inducing sepsis in mice using the Western Reserve strain of vaccinia virus, subjecting the mice to treatment with HU, and then identifying a correlation between mouse survival and ANC, body weights, and a correlation between ANC and number of virus particles.
FIG. 19 illustrates photographs taken after subjecting mice with sepsis induced by the Western Reserve strain of vaccinia virus to administration of HU or G-CSF, and then performing staining of lung tissues.
FIG. 20 illustrates results obtained by observing changes in body weight and survival, following treatment with HU, in mice with sepsis induced by influenza virus.
FIG. 21 illustrates results obtained by observing changes in survival and ANC in mice with sepsis induced by influenza virus.
FIG. 22 illustrates photographs, showing levels of skin rashes and pustules, following treatment with HU, in monkeys with systemic inflammatory syndrome induced by vaccinia virus VV^{tk-}.
FIG. 23 illustrates results obtained by measuring the absolute neutrophil count (ANC) and the number of virus particles, following treatment with HU, in monkeys with systemic inflammatory syndrome induced by the Western Reserve strain of vaccinia virus.

### Best Mode for Carrying out the Invention

Hereinafter, the present invention will be described in detail.

In an aspect of the present invention, there is provided a pharmaceutical composition for preventing, alleviating, or treating systemic inflammatory response syndrome (SIRS) or sepsis, comprising, as an active ingredient, a compound of Formula 1 (hydroxyurea) or a pharmaceutically acceptable salt thereof. The pharmaceutical composition can be effectively used for septic shock as well as sepsis.

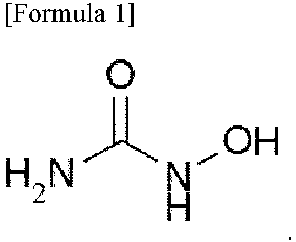

The hydroxyurea is known as an anticancer agent that inhibits DNA synthesis; however, the exact mechanism thereof is not elucidated. The hydroxyurea may be included in the pharmaceutical composition in the form of a commercialized drug that contains hydroxyurea. Examples of the commercialized drug that contains hydroxyurea may include, but are not limited to, Hydroxyurea^{®}, Hydrea^{®}, Droxia^{™}, Mylocel^{™}, Siklos^{®}, and Hydrine^{®} cap.

In addition, the pharmaceutical composition may be administered by injection, and may have a formulation for intravenous administration, for example, a liquid formulation.

Here, the hydroxyurea may be administered at a dose of 0.1 mg/kg/day to 90 mg/kg/day. Specifically, the hydroxyurea may be administered at a dose of 0.1 mg/kg/day to 90 mg/kg/day, 1 mg/kg/day to 80 mg/kg/day, 5 mg/kg/day to 70 mg/kg /day, 10 mg/kg/day to 60 mg/kg/day, or 20 mg/kg/day to 50 mg/kg/day.

In particular, it is preferable that the hydroxyurea be continuously maintained at 10 uM to 500 uM, 50 uM to 400 uM, 80 uM to 300 uM, 100 uM to 200 uM, or 120 uM to 150 uM in blood. Here, a blood concentration level of the hydroxyurea may be appropriately determined depending on the severity of systemic inflammatory response syndrome or sepsis and the patient's response rate.

As used herein, the term "systemic inflammatory response syndrome" refers to a condition in which a severe inflammatory response occurs throughout the body. A case where two or more of the following symptoms are observed is defined as systemic inflammatory response syndrome: hyperthermia with a body temperature of 38°C or higher or hypothermia with a body temperature of 36°C or lower, increased respiratory rate of over 24 breaths per minute (tachypnea), heart rate of over 90 beats per minute (tachycardia), and highly elevated or decreased white blood cell count on blood test. The systemic inflammatory response syndrome may be accompanied by an inflammatory response that occurs upon exposure to a bacterium or a virus. In addition, a systemic inflammatory response may also be induced by trauma or burns. In particular, in a case where this systemic inflammatory response syndrome is caused by microbial infection, it is called sepsis.

The systemic inflammatory response syndrome may be accompanied by an inflammatory response that occurs upon exposure to a virus, and the virus may be at least one selected from the group consisting of influenza virus, coronavirus, adenovirus, herpes simplex virus, measles virus, lentivirus, retrovirus, cytomegalovirus, baculovirus, reovirus, adeno-associated virus, myxoma virus, vesicular stomatitis virus, poliovirus, Newcastle disease virus, parvovirus, coxsackie virus, senecavirus, vaccinia virus, and poxvirus.

The virus may be wild-type or in a mutated form. The virus may also be an oncolytic virus, for example, a recombinant vaccinia virus in which thymidine kinase (TK) gene is deleted. The oncolytic virus may be a recombinant vaccinia virus (OTS-412) in which thymidine kinase gene is deleted and into which mutated herpes simplex virus type 1 thymidine kinase gene is inserted. The oncolytic virus may be a recombinant vaccinia virus (VV^{tk-}) into which granulocyte-macrophage colony stimulating factor (GM-CSF) and β-galactosidase genes are not inserted and in which thymidine kinase gene is deleted. In addition, the oncolytic virus may be a recombinant vaccinia virus in which thymidine kinase (TK) gene is deleted and into which human GM-CSF or human G-CSF gene is inserted.

As used herein, the term "sepsis" refers to a disease in which a severe inflammatory reaction occurs throughout the body. Sepsis may be accompanied by an inflammatory response that occurs upon exposure to a bacterium or a virus. In particular, sepsis is an individual's immune system disorder caused by microbial infection and may lead to serious organ damage in an individual. According to studies on the pathophysiology of sepsis, in sepsis, immune cells such as neutrophils, macrophages, and monocytes are activated after microorganism infection. The activation of these immune cells results in increased secretion of inflammatory cytokines such as IL-1, IL-6, IL-8, and TNF-α so that the transcription factor NF-κB, which is present in a cell, is activated, thereby causing an inflammatory response throughout the body. In addition, a condition, in which mortality is higher due to occurrence of circulatory, cellular, and metabolic abnormalities caused by worsening of septic symptoms, is called septic shock. If sepsis is not treated in a timely and appropriate manner, it will lead to shock or death.

Specifically, sepsis refers to a case where symptoms in a patient with suspected or proven infection satisfy the diagnostic criteria for systemic inflammatory response syndrome. For example, systemic inflammatory response syndrome is defined as a case where two or more of the followings are satisfied: body temperature of over 38°C or under 36°C, heart rate of over 90 beats/min, respiratory rate of over 20 breaths/min or PaCO2 of under 32 mmHg, and white blood cell count of more than 12,000 cells/mm³ or less than 4,000 cells/mm³ or proportion of undifferentiated cells which is more than 10%.

The sepsis may be caused by infection with a microorganism, and the microorganism may be a bacterium, a fungus, or a virus. Here, the microorganism capable of causing sepsis may include, but is not limited to, microorganisms of Streptococcus species and Enterococcus species which belong to Gram-positive bacteria. Specifically, the sepsis-causing microorganism may be Staphylococcus pneumoniae or Staphylococcus aureus. In addition, the microorganism capable of causing sepsis may include, but is not limited to, microorganisms of Klebsiella species, Pseudomonas species, and Enterobacter species, all of which belong to Gram-negative bacteria. Specifically, the sepsis-causing microorganism may be Escherichia coli, Vibrio vulnificus, or Hemophilus influenzae. The sepsis may be caused by lipopolysaccharides present in microorganisms.

In addition, the sepsis may be accompanied by an inflammatory response that occurs upon exposure to a virus, and the virus may be at least one selected from the group consisting of influenza virus, coronavirus, adenovirus, herpes simplex virus, measles virus, lentivirus, retrovirus, cytomegalovirus, baculovirus, reovirus, adeno-associated virus, myxoma virus, vesicular stomatitis virus, poliovirus, Newcastle disease virus, parvovirus, coxsackie virus, senecavirus, vaccinia virus, and poxvirus.

The virus may be wild-type or in a mutated form. The virus may also be an oncolytic virus, for example, a recombinant vaccinia virus in which thymidine kinase (TK) gene is deleted. The oncolytic virus is as described above.

As used herein, unless otherwise specified, the term "active ingredient" refers to an ingredient that exhibits activity alone or together with an adjuvant (carrier) that is not active on its own.

The pharmaceutical composition may further comprise an antibiotic or an antiviral agent. Specifically, the pharmaceutical composition may further comprise an antibiotic. The antibiotic may be at least one selected from the group consisting of doripenem, cefepime, imipenem, meropenem, ceftazidime, ceftaroline fosamil, ceftriaxone, vancomycin, teicoplanin, cefotaxime, metronidazole, aminoglycoside-based antibiotics, and combinations thereof. However, the present invention is not limited thereto.

In addition, the pharmaceutical composition may further comprise an antiviral agent. The antiviral agent may be at least one selected from the group consisting of oseltamivir, zanamivir, peramivir, acyclovir, valacyclovir, famciclovir, trifluridine, lamivudine, telbivudine, clevudine, entecavir, adefovir, tenofovir disoproxil, tenofovir alafenamide, besifovir, ribavirin, dasabuvir, sofosbuvir, daclatasvir, asunaprevir, zidovudine, abacavir, efavirenz, etravirine, nevirapine, rilpivirine, atazanavir, darunavir, nelifavir, ritonavir, indinavir, dolutegravir, raltegravil, enfuvertide, maraviroc, and combinations thereof.

The antibiotic or antiviral agent included in the pharmaceutical composition may be administered in an amount of 0.1 mg/kg to 200 mg/kg per dose based on the body weight of an individual who receives the pharmaceutical composition. Specifically, the antibiotic or antiviral agent included in the pharmaceutical composition may be administered in an amount of 0.1 mg/kg to 200 mg/kg, 1 mg/kg to 190 mg/kg, 5 mg/kg to 180 mg/kg, 10 mg/kg to 170 mg/kg, 20 mg/kg to 160 mg/kg, or 40 mg/kg to 150 mg/kg.

The pharmaceutical composition may be formulated as an injection.

As used herein, the term "injection" refers to a sterile formulation of a medicinal product that is directly applied to the body intracutaneously or through the skin and mucous membranes and is in a form such as solution, suspension, or emulsion, or any form used by being dissolved or suspended in a solvent upon use. Specifically, the injection may be used as injection, powder for injection, infusion solution, freeze-dried injection, transplant solution, long-acting injection, solution for peritoneal dialysis, perfusion solution, dialysis solution, or the like. Specifically, the injection may be administered as a bolus or by instillation, subcutaneous injection, or intramuscular injection.

The compound in the injection may be in a form dissolved in water for injection. Here, the water for injection may be physiological saline injection, Ringer's solution, or other aqueous solvents. In addition, the compound may be dissolved in a non-aqueous solvent such as vegetable oil and used.

Here, the compound may be included in the injection at a concentration of 0.1 mg/ml to 100 mg/ml, 1 mg/ml to 90 mg/ml, 10 mg/ml to 80 mg/ml, 20 mg/ml to 70 mg/ml, 30 mg/ml to 60 mg/ml, or 40 mg/ml to 50 mg/ml.

The injection may be prepared as a physically and chemically very stable injection by adjusting the pH thereof with an aqueous acid solution or a buffer such as phosphate, which may be used for injection, in order to ensure product stability during distribution of injectable formulations. Specifically, the pharmaceutical composition may comprise water for injection. The water for injection refers to distilled water made to dissolve a solid injection or to dilute a water-soluble injection.

The pharmaceutical composition may comprise a stabilizer or a solubilizing agent. For example, the stabilizer may be sodium pyrosulfite or ethylenediaminetetraacetic acid, and the solubilizing agent may be hydrochloric acid, acetic acid, sodium hydroxide, sodium hydrogen carbonate, sodium carbonate, or potassium hydroxide.

The pharmaceutical composition may further comprise other excipients commonly used for injections.

In another aspect of the present invention, there is provided a pharmaceutical composition for preventing, alleviating, or treating neutrophilia, comprising, as an active ingredient, the compound of Formula 1 (hydroxyurea) or a pharmaceutically acceptable salt thereof.

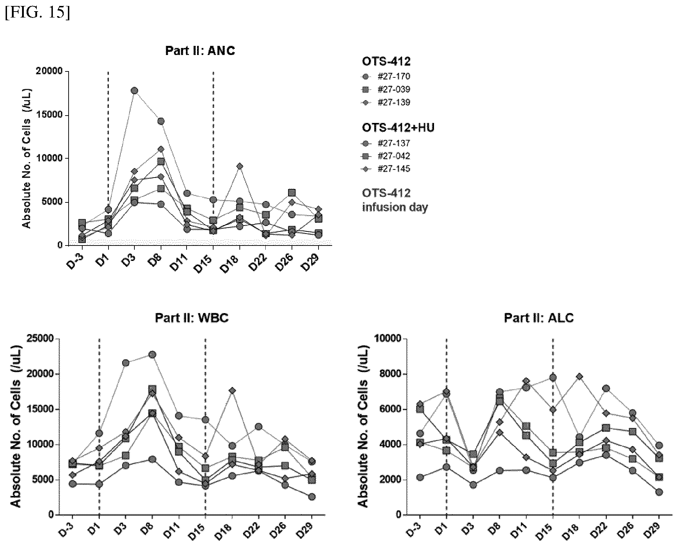

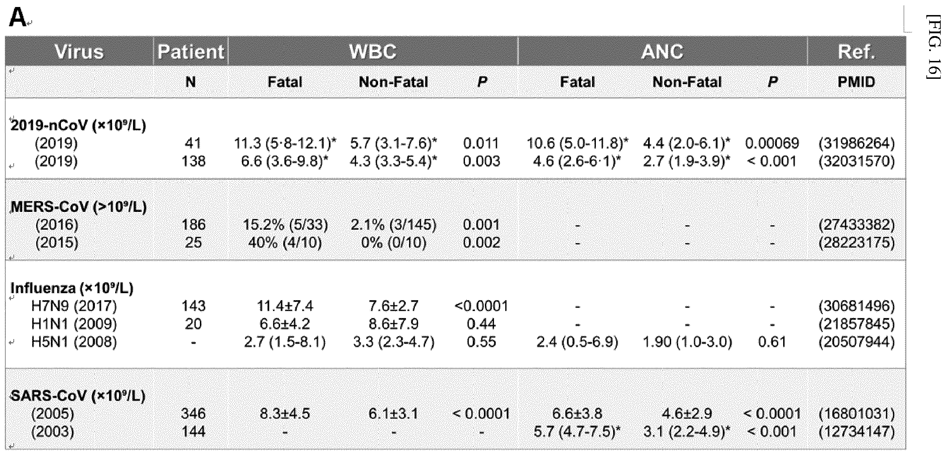

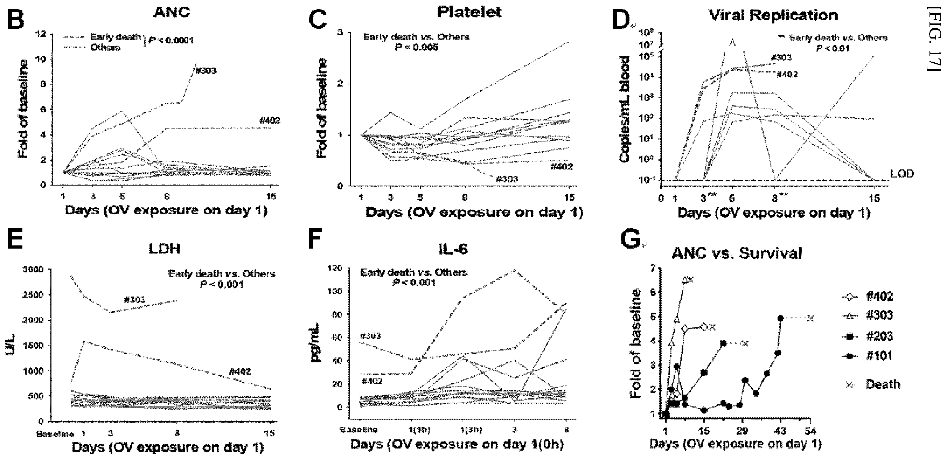

For the patients who died from infection with a pathogenic virus such as SARS-CoV-2 or were subjected to treatment in the intensive care unit (ICU), it was clinically identified that these patients had increased absolute neutrophil counts and decreased lymphocyte counts as compared with survivors and non-ICU patients (FIG. 16). In addition, it was identified that the patients who died early upon administration of an oncolytic virus (JX-594) had rapidly increased absolute neutrophil counts (FIG. 17). The present inventors have found that neutrophilia defined as an increase in absolute neutrophil count was also observed in mouse and monkey models in which sepsis or systemic inflammatory response syndrome was induced by viral infection, and that administration of the hydroxyurea to the mouse and monkey models resulted in a decrease in the increased absolute neutrophil count (FIGS. 15, 18, 21, and 23).

As used herein, the term "neutrophilia" refers to a condition in which neutrophilic granulocytes are increased above normal in peripheral blood, and such neutrophilia may develop due to acute infections, bacterial infections, malignant tumors, inflammation, tissue necrosis, myeloproliferative diseases, or the like, or even in a case where adrenaline or adrenocorticosteroid is administered. In addition, the neutrophilia may develop due to acute infections or bacterial infections, such as caused when an individual is exposed to microorganisms, in particular, viruses. Specifically, the neutrophilia may also occur when a systemic inflammatory response occurs as in systemic inflammatory syndrome or sepsis caused by exposure to a pathogenic virus such as influenza virus or coronavirus. The neutrophilia may occur upon exposure to a bacterium or a virus. In addition, the neutrophilia may develop due to exposure to viruses for therapeutic purposes, such as oncolytic viruses.

The neutrophilia occurs upon exposure to a virus, and the virus may be any one of influenza virus, coronavirus, adenovirus, herpes simplex virus, measles virus, lentivirus, retrovirus, cytomegalovirus, baculovirus, reovirus, adeno-associated virus, myxoma virus, vesicular stomatitis virus, poliovirus, Newcastle disease virus, parvovirus, coxsackie virus, senecavirus, vaccinia virus, and poxvirus.

The virus may be wild-type or in a mutated form. The virus may also be an oncolytic virus, for example, a recombinant vaccinia virus in which thymidine kinase (TK) gene is deleted. The oncolytic virus is as described above.

The pharmaceutical composition for alleviating or treating neutrophilia according to the present invention may also further comprise an antibiotic or an antiviral agent as exemplified above.

The pharmaceutical composition for alleviating neutrophilia according to the present invention may also be formulated as an injection. The injection, and the form and concentration of the compound in the injection are as described above.

The pharmaceutical composition may comprise a stabilizer or a solubilizing agent. For example, the stabilizer may be sodium pyrosulfite or ethylenediaminetetraacetic acid, and the solubilizing agent may be hydrochloric acid, acetic acid, sodium hydroxide, sodium hydrogen carbonate, sodium carbonate, or potassium hydroxide.

The pharmaceutical composition may further comprise other excipients commonly used for injections.

In yet another aspect of the present invention, the compound of Formula 1 or a pharmaceutically acceptable salt thereof may be used together with a second active ingredient such as an antibiotic or an antiviral agent. For example, there is provided a kit for preventing, alleviating, or treating systemic inflammatory response syndrome, sepsis, or neutrophilia, comprising a first composition that includes, as an active ingredient, the compound of Formula 1 or a pharmaceutically acceptable salt thereof; and a second composition that includes, as an active ingredient, an antibiotic or an antiviral agent:

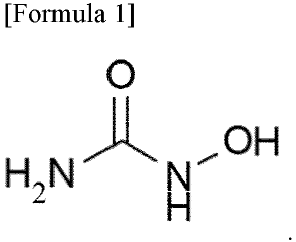

The first composition and the second composition may be administered in combination simultaneously, sequentially, or in reverse order. Specifically, the first composition and the second composition may be administered simultaneously. In addition, the first composition may be administered first, followed by the second composition. Furthermore, the second composition may be administered first, followed by the first composition. In addition, the second composition may be administered first, followed by the first composition, and then the second composition again.

The first composition and the second composition may be administered to a human or other mammals who are suffering from or suspected of having systemic inflammatory response syndrome, sepsis, or neutrophilia. In addition, the first composition and the second composition may be in the form of injections that can be administered intravenously, intramuscularly, or subcutaneously.

Each of the first composition and the second composition may be prepared as a physically and chemically very stable injection by adjusting the pH thereof with an aqueous acid solution or a buffer such as phosphate, which may be used for injection, in order to ensure product stability during distribution of injectable formulations. Specifically, each of the first composition and the second composition may comprise water for injection. The water for injection refers to distilled water made to dissolve a solid injection or to dilute a water-soluble injection.

Each of the first composition and the second composition may comprise a stabilizer or a solubilizing agent. For example, the stabilizer may be sodium pyrosulfite or ethylenediaminetetraacetic acid, and the solubilizing agent may be hydrochloric acid, acetic acid, sodium hydroxide, sodium hydrogen carbonate, sodium carbonate, or potassium hydroxide. The compositions may further comprise other excipients commonly used for injections.

In still yet another aspect of the present invention, there is provided a method for preventing, alleviating, or treating systemic inflammatory response syndrome, sepsis, or neutrophilia, comprising a step of administering the above-described pharmaceutical composition to an individual. Here, the individual may be a mammal, preferably a human.

Here, the administration may be performed intravenously, intramuscularly, or intradermally. In this way, in a case where the pharmaceutical composition according to the present invention is administered to an individual suffering from systemic inflammatory response syndrome, sepsis, or neutrophilia, the pharmaceutical composition modulates immune cells, and thus can be used for the alleviation or treatment of a systemic inflammatory response.

Here, the method may further comprise a step of administering an antibiotic or an antiviral agent. Here, the antibiotic and the administration method used are as described above.

In still yet another aspect of the present invention, there is provided a use of the above-described pharmaceutical composition for the treatment of systemic inflammatory syndrome, sepsis, or neutrophilia.

In still yet another aspect of the present invention, there is provided a use of the above-described pharmaceutical composition for the manufacture of a medicament for treating systemic inflammatory syndrome, sepsis, or neutrophilia.

### Mode for the Invention

Hereinafter, preferred examples are presented to help understand the present invention. However, the following examples are only provided for easier understanding of the present invention, and the scope of the present invention is not limited by the following examples.

### Preparation Example 1. Preparation of injectable formulation

### Preparation 1.1. Preparation of injectable formulation including hydroxyurea

Injections including hydroxyurea were prepared to have the following compositions.

**[Table 1]**

| Ingredient | Example 1 | Example 2 | Example 3 | Example 4 |
|---|---|---|---|---|
| Hydroxyurea | 1000 mg | 1000 mg | 1500 mg | 2000 mg |
| Vitamin C | - | 5 mg | 10 mg | 20 mg |
| Physiological saline | q.s. | q.s. | q.s. | q.s. |
| Total (100 ml) | 100 | 100 | 100 | 100 |

### Preparation 1.2. Preparation of injectable formulation including hydroxyurea and antibiotic

Injections including hydroxyurea and an antibiotic were prepared to have the following compositions.

**[Table 2]**

| Ingredient | Example 5 | Example 6 | Example 7 |
|---|---|---|---|
| Hydroxyurea | 1000 mg | 1500 mg | 2000 mg |
| Vitamin C | 5 mg | 10 mg | 20 mg |
| Physiological saline | q.s. | q.s. | q.s. |
| Vancomycin | 250 mg | 500 mg | 750 mg |
| Total (100 ml) | 100 | 100 | 100 |

### Preparation Example 1.3. Preparation of injectable formulation including hydroxyurea and antiviral agent

Injections including hydroxyurea and an antiviral agent were prepared to have the following compositions.

**[Table 3]**

| Ingredient | Example 8 | Example 9 | Example 10 |
|---|---|---|---|
| Hydroxyurea | 1000 mg | 1500 mg | 2000 mg |
| Vitamin C | 5 mg | 10 mg | 20 mg |
| Physiological saline | q.s. | q.s. | q.s. |
| Oseltamivir | 20 mg | 30 mg | 50 mg |
| Total (100 ml) | 100 | 100 | 100 |

### Experimental Example 1. Identification of therapeutic effect of hydroxyurea in bacterial-induced sepsis model (I)

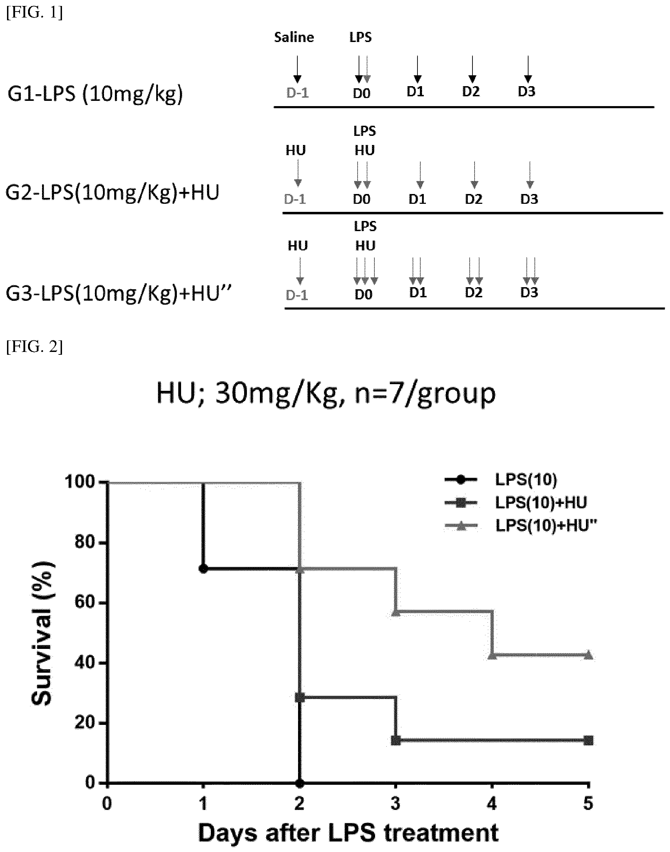

To identify whether treatment with hydroxyurea could increase survival through an immune response in a mouse model with sepsis induced by lipopolysaccharide (LPS, from Escherichia coli O55: B5L2880, Sigma), an animal experiment was performed. Each group consisted of 7 mice, and intraperitoneal administration of LPS was performed at 10 mg/kg on day 0. Two groups excluding a control group were subjected to intraperitoneal administration of hydroxyurea at 30 mg/kg once a day or twice a day for 5 days per one week starting from the day before the treatment with LPS (FIG. 1).

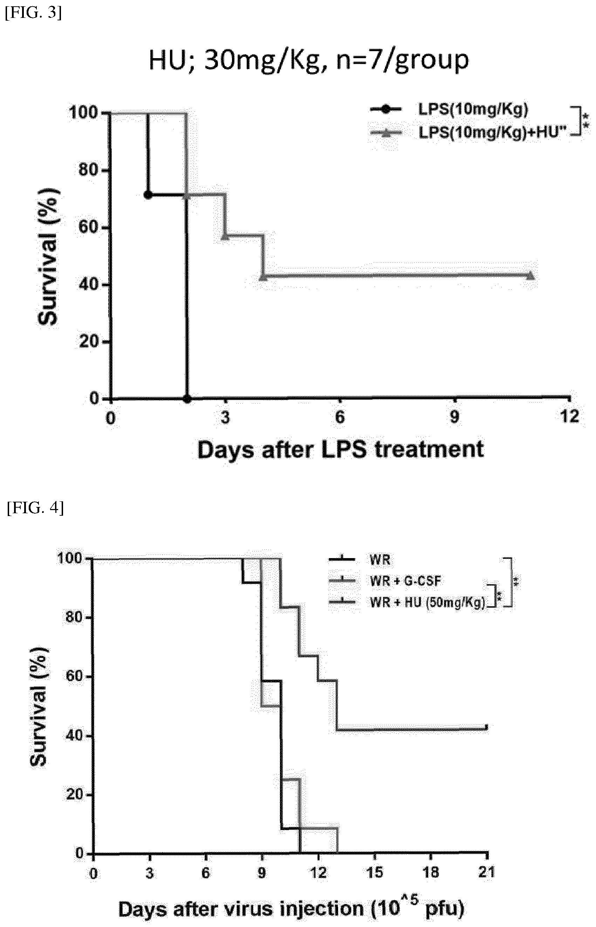

As a result, in the control group having received administration of only LPS, all seven animals died on day 2; on the other hand, the group (G2-LPS (10 mg/kg) + HU) having received administration of hydroxyurea once a day showed survival of 17% in that one animal survived until day 12, and the group (G2-LPS (10 mg/kg) + HU") having received administration of hydroxyurea twice a day showed survival of 43% in that three animals survived until day 12 (FIGS. 2 and 3).

### Experimental Example 2. Identification of therapeutic effect of hydroxyurea in bacterial-induced sepsis model (I)

### Experimental Example 2.1. Identification of increased survival caused by hydroxyurea

The Western Reserve (WR) strain of vaccinia virus is known as a virulent virus in that the virus proliferates well in a syngeneic mouse model and thus has high lethality. To identify whether hydroxyurea inhibits a systemic inflammatory response caused by viral infection and increases survival, mice were subjected to intranasal mucosal administration of the WR strain of vaccinia virus (1×10⁵ pfu or 1 × 10⁷ pfu) so that sepsis was caused by infection. Then, an animal experiment was performed. The mice were divided into a control group (WR), a group receiving G-CSF (WR + G-CSF (25 µg/kg)), and a group receiving hydroxyurea (WR + HU (50 mg/kg)). The group receiving G-CSF was used to induce increased neutrophils, and this was intended to make a comparison, in terms of immunomodulatory effect, with the group receiving hydroxyurea in which decreased neutrophils would be exhibited. Here, the WR strain of vaccinia virus as used above was a recombinant vaccinia virus in which thymidine kinase (TK) gene is deleted. For the preparation thereof, wild-type Wyeth strain (NYC Department of Health) of vaccinia virus and WR strain of vaccinia virus were purchased from the American Type Culture Collection (ATCC). For recombination, substitution of TK region in the wild-type vaccinia virus was performed using, as a vector, a shuttle plasmid that contains firefly luciferase reporter (p7.5 promoter) gene or GFP gene.

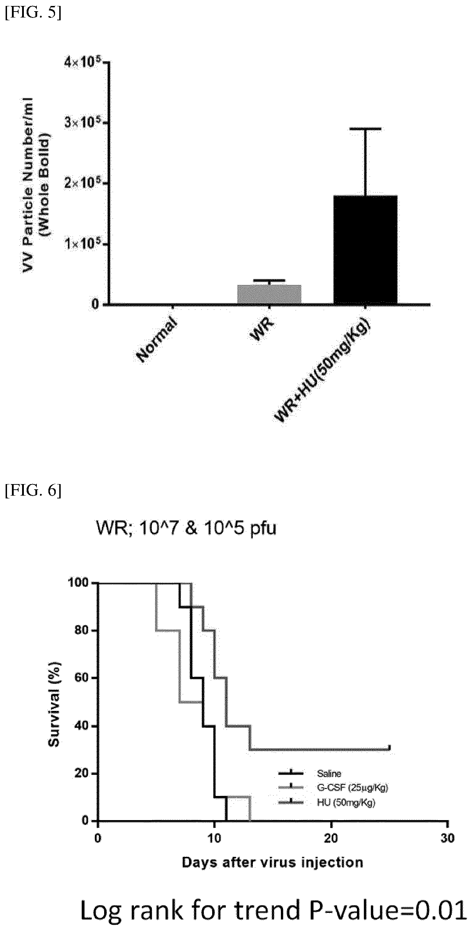

For each of the groups, survival was measured for 21 days. As a result, the group having received hydroxyurea showed survival of 40% until day 21, indicating a significant difference in survival as compared with the other two groups (p = 0.01). On the other hand, in the control group and the group having received G-CSF, all mice died within 14 days after administration of the WR strain of vaccinia virus (FIGS. 4 and 5).

Furthermore, to check whether a difference in survival seen in the group having received hydroxyurea was due to a difference in toxicity caused by the virus, the blood was collected from the mice of the group having received hydroxyurea and the number of virus particles was measured.

As a result, in the group having received hydroxyurea, the highest number of virus particles was observed. From the above results showing that despite promoted virus proliferation, the highest survival was observed due to decreased neutrophil count upon administration of hydroxyurea, it was identified that survival could be increased due to immune modulation rather than toxicity caused by the virus (FIG. 6).

### Experimental Example 2.2. Identification of effect of hydroxyurea on immune system

Regarding the mouse survival, a relationship between virus proliferation and neutrophil infiltration was determined through histological analysis. The mice were divided into a control group (WR), a group receiving G-CSF (WR + G-CSF (25 µg/kg)), and a group receiving hydroxyurea (WR + HU (50 mg/kg)) in the same manner as in Experimental Example 2.1, and the conditions set were the same except for intranasal mucosal administration of the wild-type WR strain of vaccinia virus (1×10⁷ pfu). The mice were sacrificed upon death or immediately before death, and liver and lung tissues were collected. One normal mouse having not received the virus was also sacrificed and used as a negative control group.

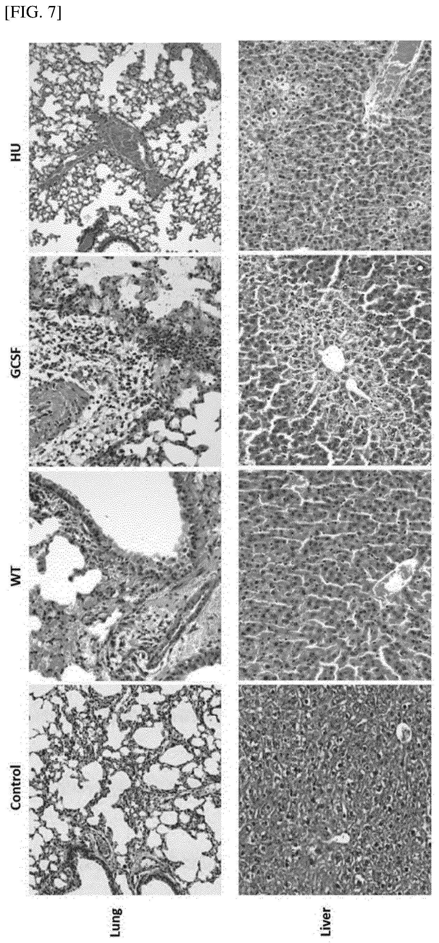

Tissue staining (H&E) was performed. As a result, slight amounts of transudate and exudate were observed in the alveoli of the mice in all groups except the negative control group, and ischemic changes were observed in zone 3 of the liver tissue (FIG. 7). Specifically, as compared with the negative control group, severity in leakage and exudate levels observed in the alveoli and ischemic change level in the liver tissue was in the order of the group having received hydroxyurea, the group having received G-CSF, and the control group.

These results suggest that the toxicity caused by the wild-type WR strain of vaccinia virus was more likely to be due to changes in hemodynamics such as decreased neutrophil count in blood rather than cytotoxicity induced by the virus proliferation itself, and well explain why HU-induced inhibition of a systemic inflammatory response is important for acute immunotoxicity caused by an oncolytic virus (FIG. 17).

### Experimental Example 3. Identification of therapeutic effect of hydroxyurea in influenza virus-induced sepsis model (I)

### Experimental Example 3.1. Identification of increased survival caused by hydroxyurea

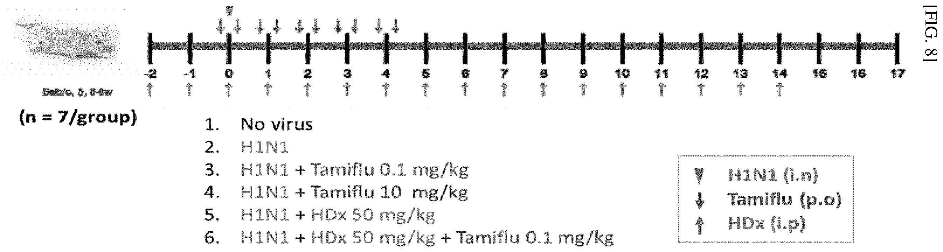

42 mice (male, BALB/c) were divided into 6 groups (n = 7/group), and the groups except for the control group were subjected to intranasal administration of H1N1 influenza A (swine flu, PR8 strain, 3× mLD50(50% mouse Lethal Dose)) virus for infection. The three groups infected with the influenza A virus were subjected to administration of Tamiflu (oseltamivir), which is known as a representative antiviral agent, and hydroxyurea alone or in combination. Tamiflu was administered at a concentration of 0.1 mg/kg or 10 mg/kg, and the hydroxyurea was administered at a concentration of 50 mg/kg (FIG. 8).

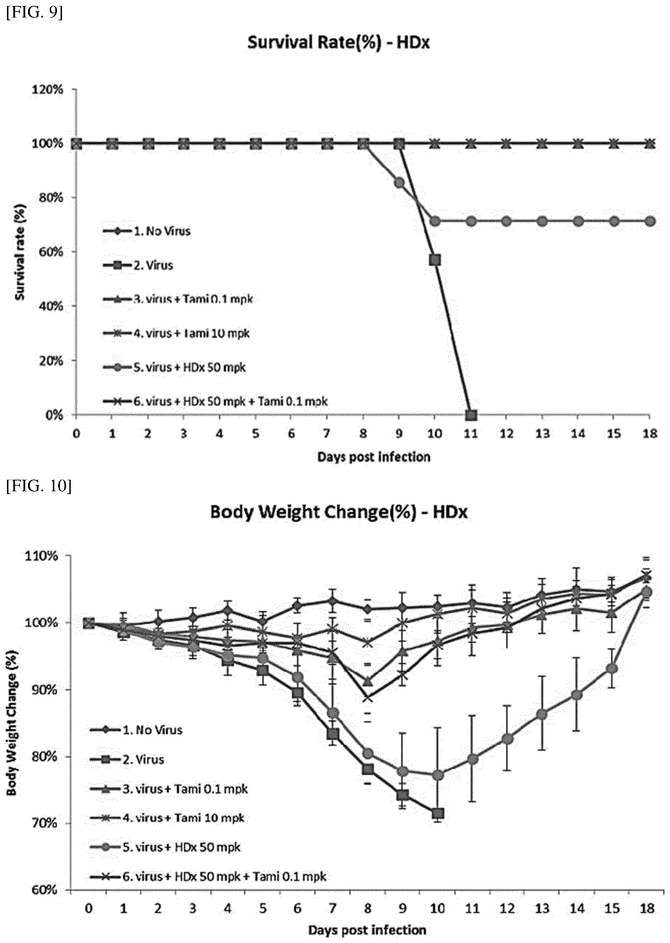

As a result, all mice of the group treated with only the influenza A virus died on day 11, whereas the mice of the three groups treated with Tamiflu survived regardless of the concentration. In the group (group HD) treated with only the hydroxyurea, two mice died on days 9 and 10, respectively; however, the remaining 5 mice all survived (FIG. 9).

In addition, body weights of the mice of each group were measured daily for 18 days. As a result, the mice of the group treated with only the influenza A virus showed an about 30% decrease in body weight and then died, and the mice of the other groups showed a decrease in body weight within 10%, and then recovered and increased back to the initial body weight (FIG. 10).

### Experimental Example 3.2. Identification of mechanism of action of hydroxyurea

To identify which mechanism results in a survival-increasing effect of hydroxyurea in an influenza virus-induced sepsis model and how such a mechanism is different from the mechanism of an antiviral agent, cells were infected *in vitro* with three influenza viruses (H1N1 PR8, H3N2 HongKong, Lee), and then treated with three different antiviral agents (ribavirin, amantadine (AMT), and oseltamivir carboxylate) as well as hydroxyurea. Then, comparison of the antiviral effects was performed.

Specifically, MDCK cells were incubated in a 96-well-plate, infected with each of three influenza viruses (H1N1 PR8, H3N2 HongKong, Lee) at 0.001 pfu/cell, and then incubated in a serum-free condition at a temperature of 33°C to 35°C. After 1 hour, washing with PBS was performed, and addition of MEM and TPCK-trypsin (2 µg/ml, Sigma) was performed. After 72 hours, cell viability against the viruses was measured by MTT assay.

As a result, the CC₅₀ of hydroxyurea was measured to be 1,561 µg/ml; however, it was identified that hydroxyurea did not show any antiviral effect on influenza A (H1N1 PR8, H3N2 HongKong) and B (Lee) viruses up to the concentration of 1,561 µg/ml. On the other hand, Tamiflu (OSV-C) clearly showed a specific antiviral effect on all three viruses, and the next clear effect was observed in the order of ribavirin and AMT.

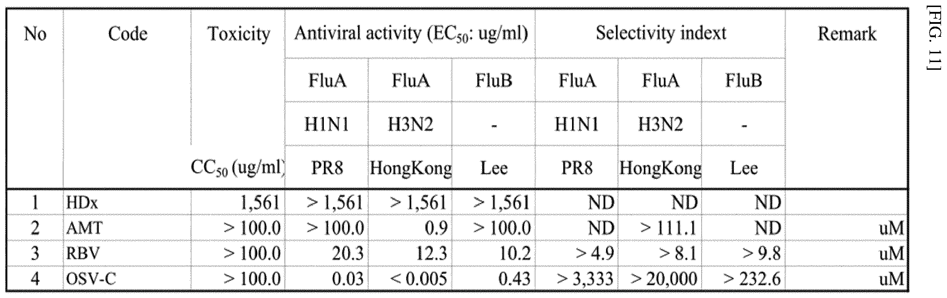

From these results, it can be identified that a survival-increasing effect of hydroxyurea in an influenza virus-induced sepsis model is not due to inhibition of viral infection, and it can be seen that such an effect is achieved by a mechanism different from the mechanism by which antiviral agents such as Tamiflu increase survival. The mice treated with only the hydroxyurea showed a decrease in body weight until day 9, similarly to the mice treated with only the virus. Then, however, all mice, which rapidly recovered and survived, returned to a healthy condition on day 18 similarly to the mice treated with Tamiflu and the control mice. This reveals that lethality caused by viral infection can decrease due to a neutrophil-modulating effect of hydroxyurea (FIG. 11).

### Experimental Example 4. Identification of inhibited systemic inflammatory response caused by hydroxyurea in monkey model (I)

Male cynomolgus monkeys (n = 3) aged 45 to 55 months were subjected to systemic administration of the WR strain of vaccinia virus (1×10⁸ pfu) in Example 2 or the WR strain of vaccinia virus and hydroxyurea (WR: 1×10⁸ pfu, HU: 80 mg/kg/day and 30 mg/kg/day). Then, toxicity was evaluated by measuring levels of systemic inflammation and pustules, body temperature, body weight, and the like.

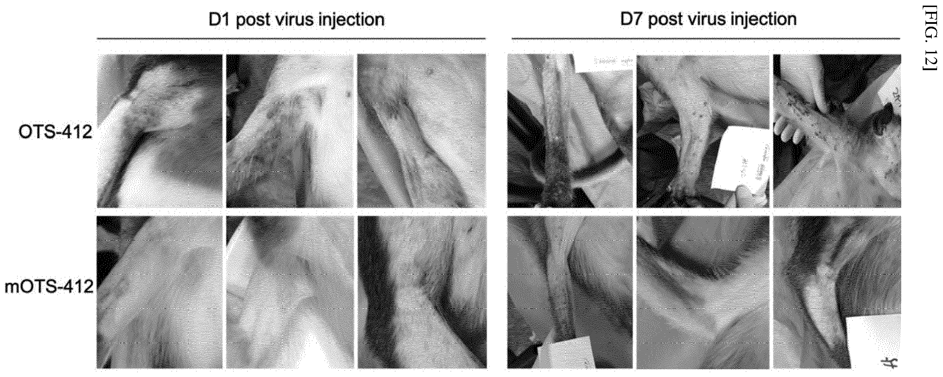

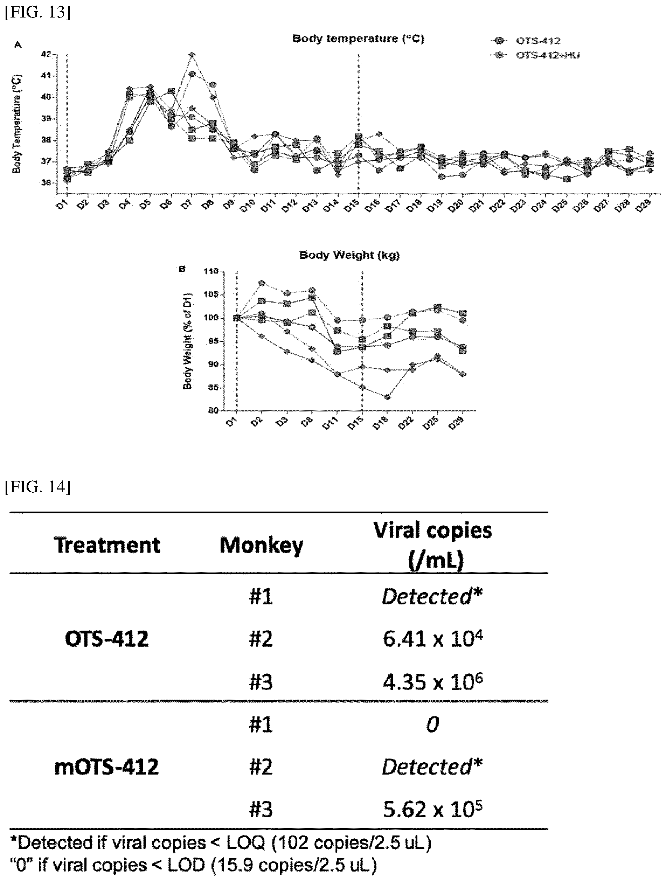

The group having received co-administration of the WR strain of vaccinia virus and hydroxyurea showed remarkably weakened systemic inflammation and inhibition of skin rash lesions such as pustules, as compared with the group having received only the WR strain of vaccinia virus (FIG. 12). The group having received only the virus showed an increase in body temperature, whereas the group having received co-administration did not show any significant increase in body temperature; and there was no difference in body weight loss between the two groups (FIG. 13).

Furthermore, the blood was collected from the monkeys of the group having received co-administration, and the number of virus particles, the absolute neutrophil count (ANC), the whole blood cell (WBC) count, and the absolute lymphocyte count (ALC) were measured.

As a result, for the group (OTS-412) having received only the virus and the group (mOTS-412) having received co-administration, there was no significant difference, in terms of number of virus particles in blood, between the two groups on day 8 after the virus administration (FIG. 14). In addition, it was identified that the absolute neutrophil count, the white blood cell count, and the absolute lymphocyte count all decreased on average and remained stable (FIG. 15).

### Reference Example 1. Review of neutrophilia caused by virus infection

A correlation between beneficial and detrimental aspects of neutrophilia caused by viral infection is still controversial. Clinical features of patients infected with SARS-CoV-2 showed an increase in absolute neutrophil count and a decrease in lymphocyte count in non-survivors and ICU patients as compared with survivors and non-ICU patients. Infection studies using other viruses also showed similar results (FIG. 16).

### Reference Example 2. Review of changes of plasma in patients upon administration of oncolytic virus

FIG. 17 illustrates results obtained by analyzing blood samples of patients who died early after administration of an oncolytic virus (JX-594). Here, it can be seen that the patients who died early had a rapidly increased ANC, showing a pathological condition related to neutrophilia.

### Experimental Example 5. Identification of therapeutic effect of hydroxyurea in bacterial-induced sepsis model (II)

72 BALB/c mice (approximately 8 weeks old) were divided into 2 major groups (n = 36/group) and subjected on day 0 to intranasal mucosal administration of the WR strain of vaccinia virus at a low dose (1×10⁵ pfu) or a high dose (1×10⁷ pfu) for infection. Each major group was subdivided into 3 subgroups (saline, G-CSF, or HU; n = 12/group). Administration of saline, G-CSF (25 µg/kg/day, on days 0 to 5), and hydroxyurea (50 mg/kg/day, on days 0 to 14) was all performed via intraperitoneal injection. Here, blood CBC test, virus replication test, and histological test were performed using three mice of each of the three subgroups. On days 1, 2, and 4, the mice of the major groups were sacrificed, and blood samples and lung tissues were obtained therefrom.

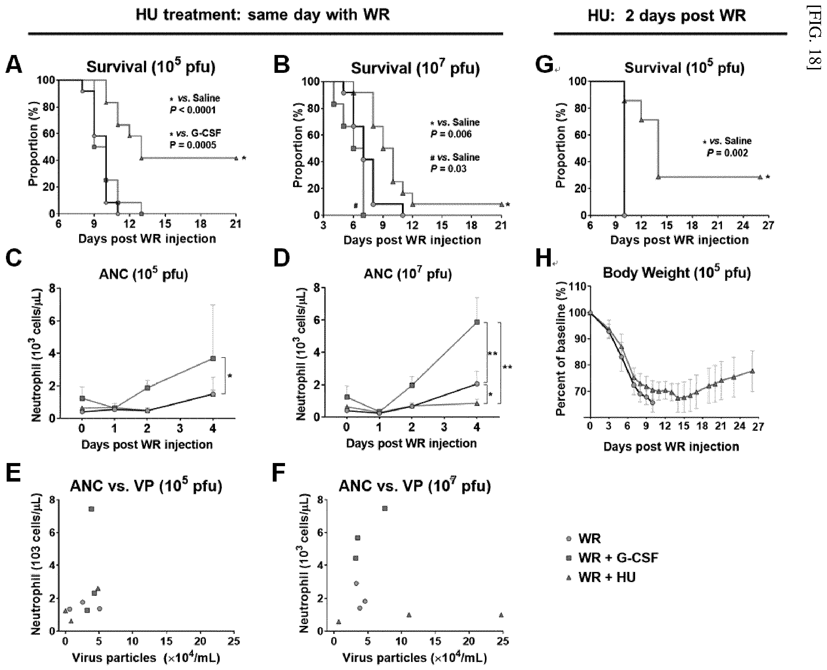

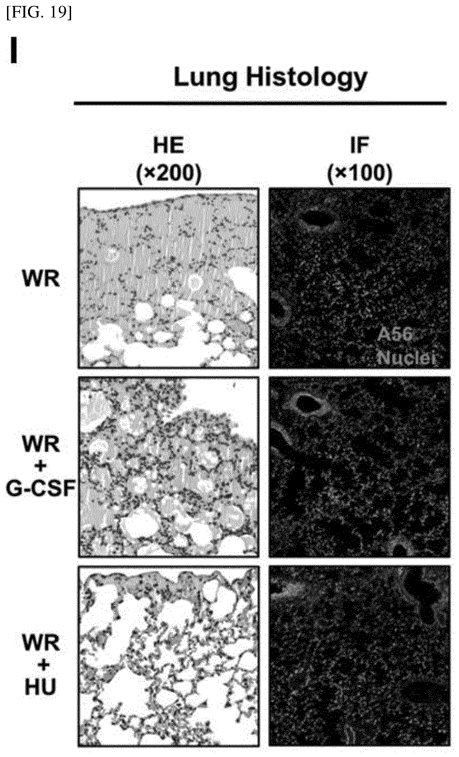

As a result, the group having received vaccinia at a low or high dose, to which hydroxyurea was administered, had increased survival (FIG. 18: A, B). In particular, the group having received vaccinia at a high dose, to which hydroxyurea was administered, also had a decreased absolute neutrophil count (ANC) along with increased survival. On the other hand, the group having received vaccinia at a low or high dose, to which G-CSF that promotes neutrophil proliferation was administered, had a remarkably increased absolute neutrophil count along with decreased survival (FIG. 19: C, D). On the other hand, there was no significant correlation between ANC and number of virus particles (VP) in the group having received vaccinia at a low or high dose (FIG. 18: E, F).

Meanwhile, additionally, 14 mice were divided into 2 groups (7 mice/group), which are the groups having post-treatment with hydroxyurea, and subjected to intranasal mucosal administration of the WR strain of vaccinia virus (1×10⁵ pfu) for infection. After 2 days, only one group was subjected to treatment with hydroxyurea (80 mg/kg/day, on days 2 to 14).

As a result, a protective effect of HU in virus therapy was observed even in a case where HU was administered 2 days after virus infection (FIG. 18: G, H).

In addition, the histological test showed that lung edema was observed in the lung tissue of the mice of the group treated with the WR strain of vaccinia virus and G-CSF rather than the lung tissue of the mice of the group treated with only the WR strain of vaccinia virus. From these results, it was identified that the cause of septic shock was other factors such as pro-inflammatory cytokines rather than pathogen-induced cytotoxicity.

On the contrary, an improved aspect was identified in the lung tissue of the mice of the group treated with the WR strain of vaccinia virus and hydroxyurea, as compared with the other groups (FIG. 19, right). In addition, infection due to the route of administration was checked by immunofluorescence staining for protein A56. As a result, infection occurred abundantly in the bronchi, and there was no significant difference between the three groups (FIG. 19, left).

### Experimental Example 6. Identification of therapeutic effect of hydroxyurea in influenza virus-induced sepsis model (II)

Twenty-eight BALB/c mice were divided into 4 groups (n = 7/group). First, the first group was set as a negative control group with no virus infection, and the second to fourth groups were subjected to intranasal mucosal administration of H1N1 influenza A virus (maPR8, 3× mLD50(50% mouse Lethal Dose)) on day 0. The seco nd group was set as a positive control group with no drug treatment, and the third and fourth groups were subjected to treatment with oseltamivir phosphate (OSV-P, 0.1 mg/kg, b.i.d., on days 0 to 4) and hydroxyurea (50 mg/kg, q.d., on days -2 to 14), respectively. Body weight and survival were measured until day 20 at 5-day intervals.

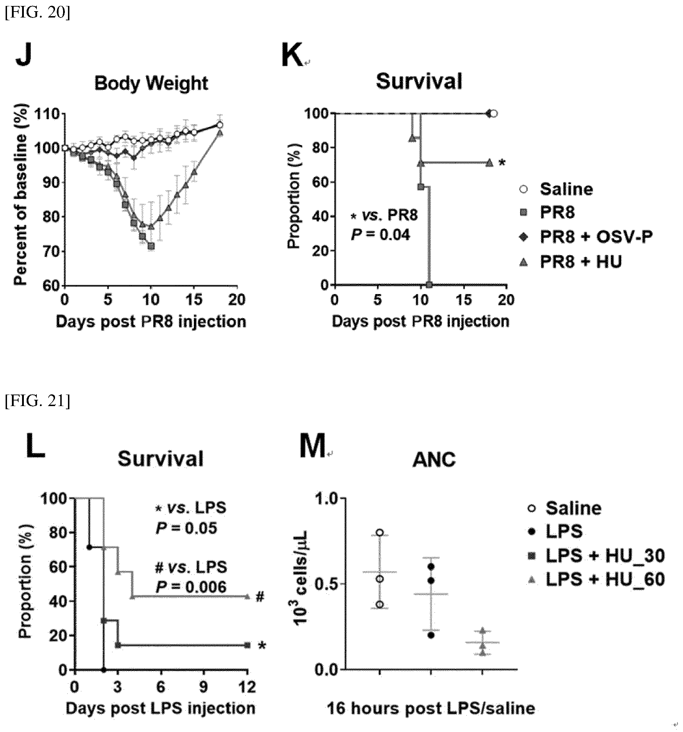

As a result, it was identified that the fourth group having received hydroxyurea had increased survival. Specifically, the mice of the second group, which is a positive control group, all died while showing a body weight loss until day 10, whereas the mice of the fourth group showed a body weight loss until day 10 and recovered to the initial weight on day 20 (70% of the mice of the fourth group survived) (FIG. 20).

### Experimental Example 7. Identification of therapeutic effect of hydroxyurea in bacterial-induced sepsis model (II)

Twenty-one BALB/c mice (about 8 weeks old) were divided into 3 groups (LPS, LPS + HU_30, LPS + HU_60; n = 7/group). All mice received intraperitoneal administration of LPS at 10 mg/kg on day 0. The mice of the group (LPS + HU_30) received hydroxyurea (30 mg/kg/day, from day -1 to day 3) once a day, and the mice of the group (LPS + HU_60) received hydroxyurea (60 mg/kg/day, from day -1 to day 3) twice a day. Survival of the mice of each group was measured until day 12 at 3-day intervals, and blood samples were collected on day 12 to check the absolute neutrophil count in blood.

As a result, all mice of the group having received only LPS died within 24 to 48 hours. On the other hand, the mice of the groups (LPS + HU_30 and LPS + HU_60) had remarkably increased survival. In addition, the mice of the groups (LPS + HU_30 and LPS + HU_60) had a decreased ANC (FIG. 21).

### Experimental Example 8. Identification of inhibited systemic inflammatory response caused by hydroxyurea in monkey model (II)

Male cynomolgus monkeys (n = 3) aged 45 to 55 months were subjected to systemic administration of only VV^{tk-} (1×10⁸ pfu), which induces a systemic inflammatory response, or both VV^{tk-} and hydroxyurea (VV^{tk-}: 1×10⁸ pfu, HU: 80 mg/kg/day and 30 mg/kg/day). Then, toxicity was evaluated by measuring levels of systemic inflammation and pustules, body temperature, body weight, and the like.

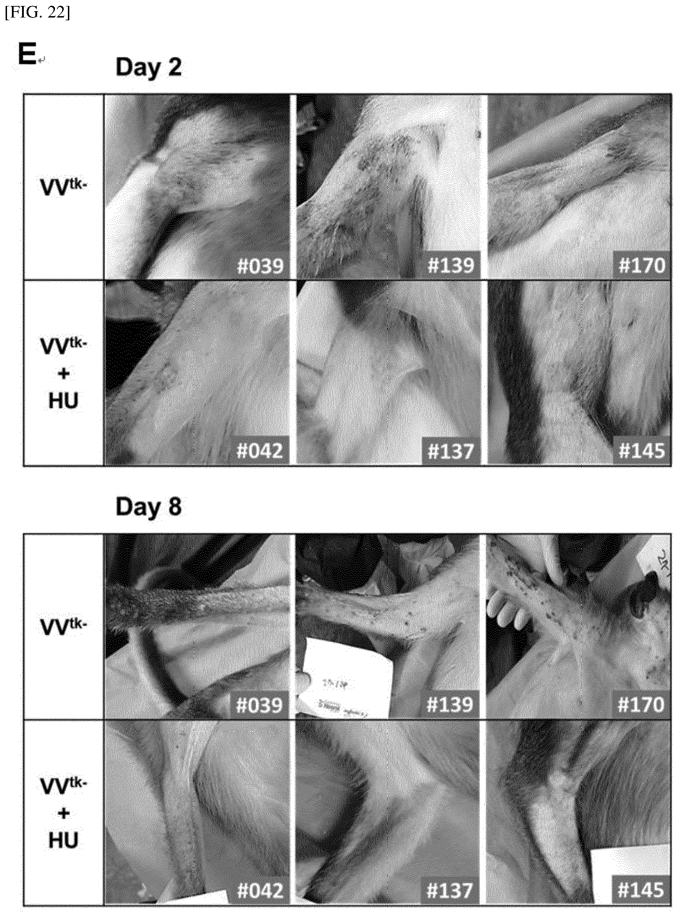

The group having received co-administration of the vaccinia virus VV^{tk-} and hydroxyurea showed remarkably weakened systemic inflammation and inhibition of skin rash lesions such as pustules, as compared with the group having received only VV^{tk-} (FIG. 22).

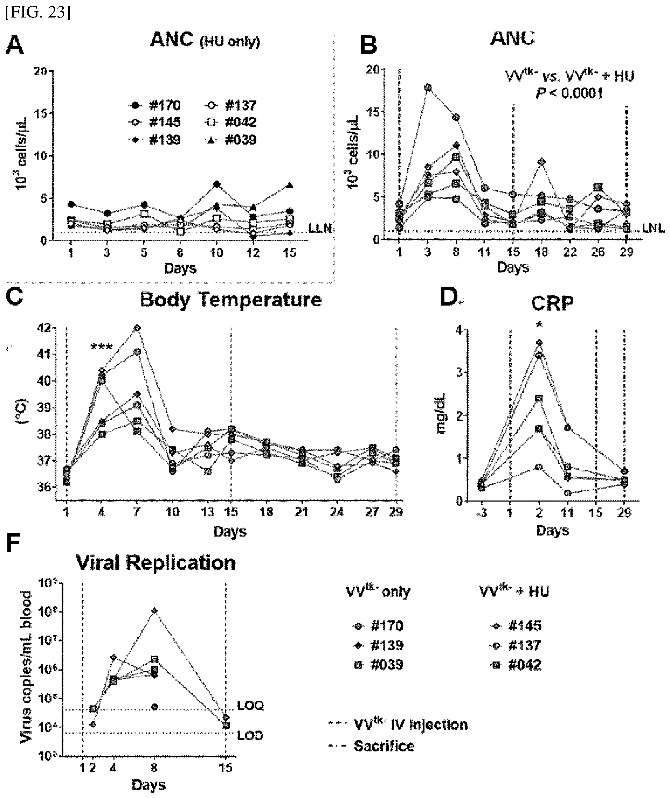

In addition, the group having received only the virus showed an increase in body temperature, whereas the group having received co-administration did not show any significant increase in body temperature; and there was no difference in body weight loss between the two groups. In addition, it was identified that both the absolute neutrophil count and the number of virus particles decreased on average and remained stable (FIG. 23).

## Claims

1. A pharmaceutical composition for preventing, alleviating, or treating systemic inflammatory response syndrome (SIRS), comprising as an active ingredient:
a compound of Formula 1 or a pharmaceutically acceptable salt thereof,

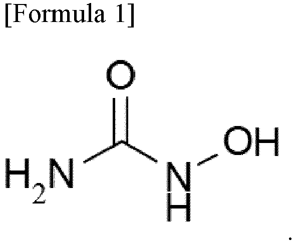

2. The pharmaceutical composition of claim 1, wherein the systemic inflammatory response syndrome is accompanied by an inflammatory response that occurs upon exposure to a bacteria or a virus.

3. The pharmaceutical composition of claim 2, wherein the systemic inflammatory response syndrome is accompanied by an inflammatory response that occurs upon exposure to a virus, and the virus is at least one selected from the group consisting of influenza virus, coronavirus, adenovirus, herpes simplex virus, measles virus, lentivirus, retrovirus, cytomegalovirus, baculovirus, reovirus, adeno-associated virus, myxoma virus, vesicular stomatitis virus, poliovirus, Newcastle disease virus, parvovirus, coxsackie virus, senecavirus, vaccinia virus, and poxvirus.

4. The pharmaceutical composition of claim 2, wherein the virus is an oncolytic virus.

5. The pharmaceutical composition of claim 1, further comprising:
an antibiotic or an antiviral agent.

6. The pharmaceutical composition of claim 5, wherein the antibiotic is at least one selected from the group consisting of doripenem, cefepime, imipenem, meropenem, ceftazidime, ceftaroline fosamil, ceftriaxone, vancomycin, teicoplanin, cefotaxime, metronidazole, aminoglycoside-based antibiotics, and combinations thereof.

7. The pharmaceutical composition of claim 5, wherein the antiviral agent is at least one selected from the group consisting of oseltamivir, zanamivir, peramivir, acyclovir, valacyclovir, famciclovir, trifluridine, lamivudine, telbivudine, clevudine, entecavir, adefovir, tenofovir disoproxil, tenofovir alafenamide, besifovir, ribavirin, dasabuvir, sofosbuvir, daclatasvir, asunaprevir, zidovudine, abacavir, efavirenz, etravirine, nevirapine, rilpivirine, atazanavir, darunavir, nelifavir, ritonavir, indinavir, dolutegravir, raltegravil, enfuvertide, maraviroc, and combinations thereof.

8. The pharmaceutical composition of claim 1, wherein the pharmaceutical composition is formulated as an injection.

9. A pharmaceutical composition for preventing, alleviating, or treating sepsis, comprising as an active ingredient:
a compound of Formula 1 or a pharmaceutically acceptable salt thereof,

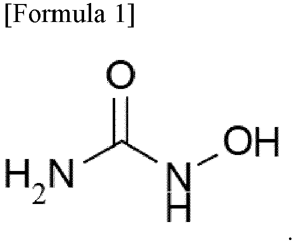

10. The pharmaceutical composition of claim 9, wherein the sepsis is accompanied by an inflammatory response that occurs upon exposure to a bacteria or a virus.

11. The pharmaceutical composition of claim 10, wherein the sepsis is accompanied by an inflammatory response that occurs upon exposure to a virus, and the virus is at least one selected from the group consisting of influenza virus, coronavirus, adenovirus, herpes simplex virus, measles virus, lentivirus, retrovirus, cytomegalovirus, baculovirus, reovirus, adeno-associated virus, myxoma virus, vesicular stomatitis virus, poliovirus, Newcastle disease virus, parvovirus, coxsackie virus, senecavirus, vaccinia virus, and poxvirus.

12. The pharmaceutical composition of claim 10, wherein the virus is an oncolytic virus.

13. The pharmaceutical composition of claim 10, further comprising:
an antibiotic or an antiviral agent.

14. The pharmaceutical composition of claim 13, wherein the antibiotic is at least one selected from the group consisting of doripenem, cefepime, imipenem, meropenem, ceftazidime, ceftaroline fosamil, ceftriaxone, vancomycin, teicoplanin, cefotaxime, metronidazole, aminoglycoside-based antibiotics, and combinations thereof.

15. The pharmaceutical composition of claim 13, wherein the antiviral agent is at least one selected from the group consisting of oseltamivir, zanamivir, peramivir, acyclovir, valacyclovir, famciclovir, trifluridine, lamivudine, telbivudine, clevudine, entecavir, adefovir, tenofovir disoproxil, tenofovir alafenamide, besifovir, ribavirin, dasabuvir, sofosbuvir, daclatasvir, asunaprevir, zidovudine, abacavir, efavirenz, etravirine, nevirapine, rilpivirine, atazanavir, darunavir, nelifavir, ritonavir, indinavir, dolutegravir, raltegravil, enfuvertide, maraviroc, and combinations thereof.

16. The pharmaceutical composition of claim 10, wherein the pharmaceutical composition is formulated as an injection.

17. A pharmaceutical composition for preventing, alleviating, or treating neutrophilia, comprising as an active ingredient:
a compound of Formula 1 or a pharmaceutically acceptable salt thereof,

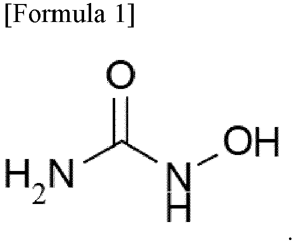

18. The pharmaceutical composition of claim 17, wherein the neutrophilia occurs upon exposure to a bacterium or a virus.

19. The pharmaceutical composition of claim 18, wherein the neutrophilia occurs upon exposure to a virus, and the virus is at least one selected from the group consisting of influenza virus, coronavirus, adenovirus, herpes simplex virus, measles virus, lentivirus, retrovirus, cytomegalovirus, baculovirus, reovirus, adeno-associated virus, myxoma virus, vesicular stomatitis virus, poliovirus, Newcastle disease virus, parvovirus, coxsackie virus, senecavirus, vaccinia virus, and poxvirus.

20. The pharmaceutical composition of claim 18, wherein the virus is an oncolytic virus.

21. The pharmaceutical composition of claim 17, further comprising:
an antibiotic or an antiviral agent.

22. The pharmaceutical composition of claim 21, wherein the antibiotic is at least one selected from the group consisting of doripenem, cefepime, imipenem, meropenem, ceftazidime, ceftaroline fosamil, ceftriaxone, vancomycin, teicoplanin, cefotaxime, metronidazole, aminoglycoside-based antibiotics, and combinations thereof.

23. The pharmaceutical composition of claim 21, wherein the antiviral agent is at least one selected from the group consisting of oseltamivir, zanamivir, peramivir, acyclovir, valacyclovir, famciclovir, trifluridine, lamivudine, telbivudine, clevudine, entecavir, adefovir, tenofovir disoproxil, tenofovir alafenamide, besifovir, ribavirin, dasabuvir, sofosbuvir, daclatasvir, asunaprevir, zidovudine, abacavir, efavirenz, etravirine, nevirapine, rilpivirine, atazanavir, darunavir, nelifavir, ritonavir, indinavir, dolutegravir, raltegravil, enfuvertide, maraviroc, and combinations thereof.

24. The pharmaceutical composition of claim 17, wherein the pharmaceutical composition is formulated as an injection.

25. A use of a compound of Formula 1 or a pharmaceutically acceptable salt thereof for the prevention, alleviation, or treatment of systemic inflammatory response syndrome (SIRS):

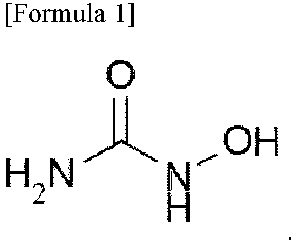

26. A use of a compound of Formula 1 or a pharmaceutically acceptable salt thereof for the prevention, alleviation, or treatment of sepsis:

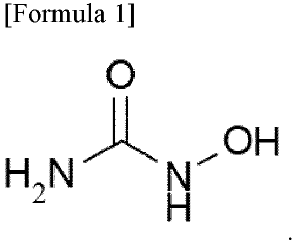

27. A use of a compound of Formula 1 or a pharmaceutically acceptable salt thereof for the prevention, alleviation, or treatment of neutrophilia:

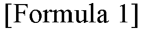

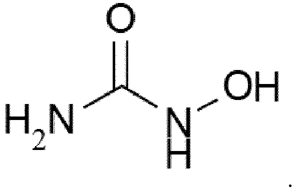

28. A method for preventing, alleviating, or treating systemic inflammatory response syndrome (SIRS), comprising:
a step of administering the pharmaceutical composition of claim 1 to an individual.

29. A method for preventing, alleviating, or treating sepsis, comprising:
a step of administering the pharmaceutical composition of claim 9 to an individual.

30. A method for preventing, alleviating, or treating neutrophilia, comprising:
a step of administering the pharmaceutical composition of claim 17 to an individual.
